# EUROPEAN PATENT SPECIFICATION

(11) **EP 2 554 157 B1**
(45) Date of publication and mention of the grant of the patent: **21.06.2017**
(21) Application number: 11762313.2
(22) Date of filing: 19.01.2011
(51) Int. Cl.: A61K 8/06, A61K 8/35, A61K 8/37, A61K 8/40, A61K 8/90, A61Q 17/04

(54) **O/W EMULSION COMPOSITION**
ÖL-WASSER-EMULSIONSZUSAMMENSETZUNG
COMPOSITION D'ÉMULSION HUILE DANS L'EAU

(30) Priority: 30.03.2010 JP 2010078039
(43) Date of publication of application: 06.02.2013
(73) Proprietor: Shiseido Co., Ltd., Tokyo 104-0061 (JP)
(72) Inventor: TAKAKURA Tomiko, Yokohama-shi Kanagawa 224-8558 (JP); KUROSAWA Takafumi, Yokohama-shi Kanagawa 224-8558 (JP); YAJIMA Isao, Yokohama-shi Kanagawa 224-8558 (JP)
(74) Representative: Lippert Stachow Patentanwälte Rechtsanwälte
(86) International application number: PCT/JP2011/050860
(87) International publication number: WO 2011/122072

(56) References cited:
- WO-A1-2010/026755
- FR-A1- 2 911 504
- JP-A- 2007 014 866
- JP-A- 2007 106 715
- JP-A- 2009 520 707
- JP-A- 2010 024 161
- US-A1- 2004 166 072

## Description

### FIELD OF THE INVENTION

The present invention relates to an oil-in-water (O/W) emulsion composition and, in particular, to an O/W emulsion composition which contains oil-soluble organic UV absorbers, and is excellent in formulation stability and feeling in use and suitable as a sunscreen cosmetic.

### BACKGROUND OF THE INVENTION

In cosmetics, UV protection ability has been often provided by incorporating organic UV absorbers or inorganic UV scatterers such as fine particle titanium oxide and fine particle zinc oxide. In recent years in particular, the effect of ultraviolet on the skin has become widely known recently, and users have become increasingly conscious of skin whitening. Thus, there is demand for a cosmetic which provides a higher UV protection ability and even a good feeling in use.

Among these cosmetics, due to the fact that an O/W emulsion composition can provide fresh and light-refreshing feeling in use while it contains oils, such emulsion composition is widely used not only in cosmetics for basic skin care such as milky lotions and creams, but also in products such as foundations and sunscreen cosmetics.

However, when a high UV protection ability is tried to be provided over a wide range of UV-B to UV-A by incorporating a large amount of inorganic UV shielding powder such as fine particle titanium oxide and fine particle zinc oxide, the finish may become whitish, or frictional or powdery feeling may be caused.

In contrast, many organic UV absorbers are generally highly polar oils and do not cause the problems such as the whitish finish and frictional or powdery feeling described above. However, they provide stickiness so much to deteriorate the fresh feeling of O/W emulsion composition when the emulsion composition is applied to skin. In addition, the emulsion stability tends to decrease. In particular, when octocrylene and ethylhexyl methoxycinnamate are used in combination as organic UV absorbers, an excellent UV protection ability can be achieved, but it has been difficult to obtain an O/W emulsion composition with a high emulsion stability and a good feeling in use.

Furthermore, 4-t-butyl-4'-methoxydibenzoylmethane is an excellent organic UV absorber having an UV absorption ability in the region extending from UV-A to UV-B, and especially in the range of UV-A. However, the compound is essentially a crystalline solid at normal temperature, and when it is intended to incorporate this compound into an O/W emulsion composition by dissolving the compound in an oil phase, there is a problem that the compound is not easily dissolved and precipitates over time.

JP 2007 106715 A discloses an O/W emulsion comprising UV absorbers ethylhexyl methoxycinnamate and 4-t-butyl-4'-methoxydibenzoylmethane and a polyoxyethylene/ polyoxyalkylene alkyl ether block polymer.

JP 2010 024161 A discloses an O/W emulsion comprising UV absorbers ethylhexyl methoxycinnamate and 4-t-butyl-4'-methoxydibenzoylmethane and a polyoxyethylene/ polyoxyalkylene alkyl ether block polymer.

US 2004/166072 A1 discloses an O/W emulsion comprising UV absorbers octocrylene, ethylhexyl methoxycinnamate and 4-t-butyl-4'-methoxydibenzoylmethane.

EP2181697 (see WO2007122822) discloses sunscreens in the form of fine o/w-emulsions comprising octocrylene, ethylhexyl methoxycinnamate, 4-t-butyl-4'-methoxydibenzoylmethane and a polyoxyehtylene/polyoxypropylene alkyl ether block polymer.

### DISCLOSURE OF THE INVENTION

### PROBLEM TO BE SOLVED BY THE INVENTION

As such, when an UV absorber is combined, high UV protection ability can be realized. However, when an emulsion composition is prepared, it is necessary to incorporate a large amount of surfactants in order for the emulsion composition to have emulsion stability. Thus, an oily feeling in use characteristic to an emulsion having an UV protection ability tends to come out.

The present invention was made in view of the above-described conventional art. An object of the invention is to provide an O/W emulsion composition which is excellent in UV protection ability, formulation stability, and non-sticky and fresh feeling in use.

### MEANS TO SOLVE THE PROBLEM

To solve the aforementioned problems, the present inventors have diligently studied and found that an O/W emulsion composition which is excellent in UV protection ability and formulation stability over time and has non-sticky and fresh feeling in use can be obtained by dissolving 4-t-butyl-4'-methoxydibenzoylmethane in the oil phase containing octocrylene and ethylhexyl methoxycinnamate, and allowing the oil phase to have a certain emulsion particle size or less with use of a specific emulsifier, thus leading to completion of the present invention.

That is, the O/W emulsion composition of the present invention is characterized by comprising the following components (a) and (b):
(a) organic UV absorbers comprising the following components (a1), (a2), and (a3):
   (a1) octocrylene,
   (a2) ethylhexyl methoxycinnamate,
   (a3) 4-t-butyl-4'-methoxydibenzoylmethane; and
(b) a polyoxyethylene/polyoxyalkylene alkyl ether block polymer represented by the following formula (1);
(Compound 1)

R₁O-(PO)m-(EO)n-H (1)

wherein R₁ is a hydrocarbon group having 16 to 18 carbon atoms; PO is an oxypropylene group, EO is an oxyethylene group, and PO and EO are added to each other in block form; and m and n respectively represent average addition mole number of PO and EO, 4≤m<70, 10≤n<70, and m< n; and
wherein average particle size of the emulsion particles comprising the component (a) is 800 nm or less, and
wherein the value of {amount of (a1) + amount of (a2)}/amount of (a3) is 2 or more.

In the O/W emulsion composition, it is preferable that the amount of component (a) is 8 mass % or more.

In the O/W emulsion composition, it is preferable that the amount of component (b) is 0.5 to 3 mass %.

In the O/W emulsion composition, it is preferable that the amount of component (a1) is 1 to 10 mass %, the amount of component (a2) is 1 to 7.5 mass %, and the amount of component (a3) is 0.5 to 5 mass %.

Also, the present invention provides a sunscreen cosmetic consisting of the O/W emulsion compositions.

### EFFECT OF THE INVENTION

4-t-butyl-4'-methoxydibenzoylmethane is dissolved in an oil phase containing octocrylene and ethylhexyl methoxycinnamate, and then the oil phase is emulsified finely with use of a specific emulsifier to be a certain emulsion particle size or less, whereby an O/W emulsion composition which shows high UV protection ability in a wide UV range and is also excellent in formulation stability and feeling in use can be obtained. The emulsion composition can contain a large amount of organic UV absorbers and is very useful for a sunscreen cosmetic.

### BEST MODE FOR CARRYING OUT THE INVENTION

The O/W emulsion composition of the present invention is characterized by comprising component (a) specific organic UV absorbers and component (b) specific polyoxyethylene/polyoxyalkylene alkyl ether block polymer. Also, in the O/W emulsion composition of the present invention, the average particle size of the emulsion particles comprising the component (a) is 800 nm or less.

In the following, each component is described in detail.

### ((a) Organic UV absorber)

Among the organic UV absorbers used in the present invention, (a1) octocrylene (chemical name: 2-ethylhexyl 2-cyano-3,3-diphenylacrylate) is an UV absorber which is in an oil state at ordinary temperature, and commercially available products such as "Uvinul N539" (manufactured by BASF) or "Parsol 340" (manufactured by DSM Nutrition Japan K.K.) can be easily used.

The amount of octocrylene can be suitably set depending on purposes. From a viewpoint of UV protection ability, solubility of solid components, the amount of octocrylene is preferably 1 mass % or more, more preferably 2 mass % or more, and particularly preferably 3 mass % or more, in the O/W emulsion composition of the present invention. On the other hand, when octocrylene is contained excessively, the feeling in use is deteriorated with stickiness and an oily feeling. Thus, the amount is preferably 10 mass % or less, more preferably 8 mass % or less, and particularly preferably 6 mass % or less, in the O/W emulsion composition of the present invention.
(a2) Ethylhexyl methoxycinnamate (octyl methoxycinnamate) is an UV absorber which is in an oil state at ordinary temperature, and commercially available products such as "Parsol MCX" (manufactured by DSM Nutrition Japan K.K.) can be easily used.
   The amount of ethylhexyl methoxycinnamate can be suitably set depending on purposes. From a viewpoint of UV protection ability, solubility of solid components, the amount of ethylhexyl methoxycinnamate is preferably 1 mass % or more, more preferably 2 mass % or more, and particularly preferably 4 mass % or more, in the O/W emulsion composition of the present invention. On the other hand, when ethylhexyl methoxycinnamate is contained excessively, the feeling in use is deteriorated with stickiness and an oily feeling. Thus, the amount is preferably 7.5 mass % or less, more preferably 7 mass % or less, and particularly preferably 6 mass % or less, in the O/W emulsion composition of the present invention.
(a3) 4-t-butyl-4'-methoxydibenzoylmethane is an UV absorber which is in an oil state at ordinary temperature, and commercially available products such as "Parsol 1789" (manufactured by DSM Nutrition Japan K.K.) can be easily used.
   The amount of 4-t-butyl-4'-methoxydibenzoylmethane can be suitably set depending on purposes. From a viewpoint of UV protection ability, the amount of 4-t-butyl-4'-methoxydibenzoylmethane is preferably 0.5 mass % or more, more preferably 1 mass % or more, and particularly preferably 1.5 mass % or more, in the O/W emulsion composition of the present invention. On the other hand, when 4-t-butyl-4'-methoxydibenzoylmethane is contained excessively, crystals are likely to be precipitated over time. Thus, the amount is preferably 5 mass % or less, more preferably 4 mass % or less, and particularly preferably 3 mass % or less, in the O/W emulsion composition of the present invention.
(a3) 4-t-butyl-4'-methoxydibenzoylmethane has a problem that the compound is not easily dissolved and is likely to be precipitated over time. However, in the present invention, an O/W emulsion composition having excellent solubility stability can be prepared by incorporating 4-t-butyl-4'-methoxydibenzoylmethane into an oil phase containing (a1) octocrylene and (a2) ethylhexyl methoxycinnamate. From the viewpoint of solubility stability, the proportion of the amounts of (a1) and (a2) with respect to the amount of (a3), that is, the value of {amount of (a1) + amount of (a2)}/amount of (a3) is preferably 2 or more, and particularly preferably 4.5 or more.

Considering an UV protection ability, the amounts of (a) organic UV absorbers are preferably 8 mass % or more, more preferably 9 mass % or more, and particularly preferably 10.5 mass % or more, in the O/W emulsion composition. In the present invention, even when such large amounts of oil-soluble organic UV absorbers are incorporated, the O/W emulsion composition excellent in formulation stability and feeling in use can be obtained.

### ((b) Polyoxyethylene/polyoxyalkylene alkyl ether block polymer)

In the present invention, as an emulsifier for the oil phase containing the component (a), it is necessary that one or more polyoxyethylene/polyoxyalkylene alkyl ether block polymers represented by the following formula (1) or (2) are used. With the use of the emulsifier, the O/W emulsion composition in which the oil phase containing the component (a) is finely and stably emulsified can be easily produced.
(Compound 1)

R₁O-(PO)m-(EO)n-H (1)

In the formula (1), R₁ is a hydrocarbon group having 16 to 18 carbon atoms, and it is preferably a saturated or unsaturated aliphatic hydrocarbon group. The examples include palmityl, stearyl, isostearyl, oleyl, and linolyl groups.

PO is an oxypropylene group, and EO is an oxyethylene group.

In the formula (1), PO and EO must bond to each other in block form. When they bond to each other in random form, the formulation stability cannot be sufficiently achieved. The addition order of propylene oxide and ethylene oxide is not particularly specified. The block includes not only two-stepwise block, but also three- or more-stepwise block.

m and n respectively represent the average addition mole number of PO and EO, 4≤m<70, 10≤n<70, and m<n.

The molecular weight of the block polymer in the formula (1) is preferably 800 or more, and more preferably 1500 or more. When the molecular weight is less than 800, the effect is low. Though the upper limit of the molecular weight cannot be specified particularly, stickiness tends to be caused as the molecular weight becomes larger. Examples of the block polymer represented by the formula (1) include Nikkol PBC44 (manufactured by Nikko Chemicals Co., Ltd.).

As even a small amount of these block polymers can emulsify finely and stably the oil phase containing the component (a), the stable O/W emulsion composition without stickiness owing to surfactants can be obtained. However, when the amount of block polymer is too small, the stable O/W emulsion composition cannot be obtained. Thus, the amount is preferably 0.5 to 3 mass %, more preferably 0.5 to 2 mass %, and particularly preferably 0.5 to 1 mass %, in the composition.

In the O/W emulsion composition of the present invention, other ingredients which can be incorporated in cosmetics can be further contained in addition to the essential components described above so far as the effect of the present invention is not affected. For examples, powders, liquid oils, solid oils, waxes, hydrocarbons, higher fatty acids, higher alcohols, esters, silicones, anionic surfactants, cationic surfactants, amphoteric surfactants, nonionic surfactants, moisturizers, water-soluble polymers, thickeners, film-forming agents, UV absorbers, UV scatterers, metal ion sequestering agents, lower alcohols, polyhydric alcohols, saccharides, amino acids, organic amines, polymer emulsions, pH adjusters, skin nutrients, vitamins, antioxidants, antioxidant aids, perfumes, and water can be suitably incorporated as necessary.

In the O/W emulsion composition of the present invention, organic UV absorbers other than the above-described components (a1) to (a3) also can be contained. Examples thereof include oily UV absorbers generally used in cosmetics. For example, triazine UV absorbers such as bis(resorcinyl)triazine and bis-ethylhexyloxyphenol methoxyphenyl triazine; octyl triazone (2,4,6-tris-[4-(2-ethylhexyloxycarbonyl)anilino]-1,3,5-triazine); benzoic acid UV absorbers such as p-aminobenzoic acid (hereinafter abbreviated as "PABA"), PABA monoglycerin ester, N, N-dipropoxy PABA ethyl ester, N, N-diethoxy PABA ethyl ester, N, N-dimethyl PABA ethyl ester, N, N-dimethyl PABA butyl ester, and N, N-dimethyl PABA ethyl ester; anthranilic acid UV absorbers such as homomenthyl-N-acetyl anthranilate; salicylic acid UV absorbers such as amyl salicylate, menthyl salicylate, homomenthyl salicylate, octyl salicylate, phenyl salicylate, benzyl salicylate, and p-isopropanol phenyl salicylate; cinnamic acid UV absorbers such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl p-methoxycinnamate (2-ethylhexyl p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glyceryl mono-2-ethylhexanoyl-dipara methoxycinnamate; benzophenone UV absorbers such as 2, 4-dihydroxybenzophenone, 2,2'-dihydroxy-4-methoxybenzophenone, 2, 2'-dihydroxy-4, 4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d, l-camphor; 3-benzylidene-d, 1-camphor; 2-phenyl-5-methyl benzoxazole; 2, 2'-hydroxy-5-methylphenyl benzotriazole; 2-(2'-hydroxy-5'-t-octylphenyl) benzotriazole; 2-(2'-hydroxy-5'-methylphenyl)benzotriazole; dibenzaladine; dianisoylmethane; 5-(3,3-dimethyl-2-norbornylidene)-3-pentane-2-one, methylene bis-benzotriazolyl tetramethylbutylphenol, and 4,4-diarylbutadiene can be listed.

The O/W emulsion composition of the present invention is such that the average particle size of the emulsion particles containing the component (a) is 800 nm or less. If the particle size exceeds 800 nm, the feeling of use is deteriorated, and a decrease in the formulation stability (separation of oils over time, the occurrence of creaming, aggregation, and precipitation) may occur.

Any emulsification method used in the present invention can be used so far as the emulsion particles can be emulsified so finely as to be 800 nm or less. The examples include a high-pressure emulsification method and a micro emulsification method using a hydrophilic solvent such as a polyhydric alcohol in the presence of a small amount of water (or in the absence of water) (see Japanese Examined Patent Publication No. S57-29213, Japanese Unexamined Patent Publication No. 2006-182724) ; however, they are not limited thereto.

Such O/W emulsion composition of the present invention is preferably a composition that the emulsion particles having the average particle size of 800 nm or less and containing octocrylene, ethylhexyl methoxycinnamate, and 4-t-butyl-4'-methoxydibenzoylmethane are dispersed in the aqueous phase which is a continuous phase, and that the oil phase is a homogenous phase in which 4-t-butyl-4'-methoxydibenzoylmethane is dissolved.

The production method is not limited in particular, and the O/W emulsion composition can be typically produced by emulsifying, with use of (b) polyoxyethylene/polyoxyalkylene alkyl ether block polymer, the oil phase in which (a1) octocrylene, (a2) ethylhexyl methoxycinnamate, and (a3) 4-t-butyl-4'-methoxydibenzoylmethane are mixed and dissolved and the aqueous phase. Here, it is suitable to have the (b) polyoxyethylene/polyoxyalkylene alkyl ether block polymer added in advance to the aqueous phase. As far as any problem is not caused in particular, other ingredients may be incorporated in the oil or aqueous phase depending on their compatibility or affinity.

The O/W emulsion composition of the present invention is applicable in various cosmetics in which sunscreen function is desired. For example, it is applicable in makeup cosmetics such as foundations and lipsticks, as well as milky lotions, creams, and pre-makeup.

### EXAMPLES

Hereinafter, the present invention will be further explained with reference to specific examples. However, the present invention is not limited by these examples. The amount is expressed in mass % unless otherwise specified. The evaluation methods used in the present invention are as follows.

### Evaluation (1): Average Emulsion Particle Size

The average particle size of emulsion particles of each sample just after production was measured with Zetasizer Nano ZS (manufactured by Sysmex Corporation).
A: Average particle size was 800 nm or less.
C: Average particle size exceeded 800 nm.

### Evaluation (2): Emulsion Stability

The appearance of each sample that had been preserved at 50 °C for one month was observed by the naked eyes and evaluated according to the following criteria.
A: There was no oil floatation or creaming.
B: There were slight oil floatation and creaming.
C: There were oil floatation and creaming.

### Evaluation (3): Feeling in Use

In 20 female panelists, each sample just after production was applied to the face by hand and evaluated according to the following criteria with the questionnaire for non-stickiness during application.
A: 16 or more panelists answered that there was no stickiness.
B: 6 or more to 15 or less panelists answered that there was no stickiness.
C: 5 panelists or less answered that there was no stickiness.

### Evaluation (4): Solubility Stability

Oils of each sample were dissolved. After they had been preserved at 0 °C for one month, the precipitate was observed by the naked eyes and evaluated according to the following criteria.
A: Crystals or insoluble matter was not precipitated.
B: Crystals and insoluble matter were precipitated slightly.
C: Crystals and insoluble matter were precipitated.

### Evaluation (5): UV Protection Ability

The UV protection ability of each sample just after production was measured with in vitro SPECTRO PHOTOMETER U-4100 (manufactured by Hitachi, Ltd.) and evaluated according to the following criteria.
A: The absorbance at 310 nm was higher than that of a reference sample showing in vivo measurement value of SPF 16.
C: The absorbance at 310 nm was lower than that of a reference sample showing in vivo measurement value of SPF 16.

### TEST EXAMPLE 1 Emulsion particle size

O/W emulsion compositions with the blending compositions shown in Table 1 were prepared by the following production method. Then, each sample was evaluated in the above-described evaluation methods (1) to (4). Meanwhile, in regard to the evaluation method (1), measurement was carried out by the method described above, and the results are indicated as specific numerical values. The result is shown in Table 1.

### •Production method 1

### (Part 1)

A portion of component (1) and components (7) and (8) were mixed, and component (6) was added gradually thereto. The mixture was emulsified. The mixture in which components (3) to (5), components (10) to (12), and component (16) were dissolved and mixed was added to the emulsion. The mixture was emulsified, and thus a Part 1 was obtained.

### (Part 2)

A portion of component (1) and components (13) to (15) were dissolved and mixed, and a mixture prepared by dissolving and mixing components (2) and (17) was added thereto. The mixture was mixed, and thus a Part 2 was obtained.

The Part 1 was uniformly dispersed in the Part 2, and a mixture of a portion of component (1) and component (9) prepared by wet dispersing was added thereto. Thus, an intended O/W emulsion composition was obtained.

### •Production method 2

### (Part 1)

Components (3) to (5), components (10) to (12), and component (16) were dissolved and mixed. The mixture and component (6) were mixed, and thus a Part 1 was obtained.

### (Part 2)

A mixture prepared by mixing a portion of component (1) and components (7) and (8) and a mixture prepared by mixing a portion of component (1) and components (13) to (15) were mixed. Then, a mixture prepared by dissolving and mixing components (2) and (17) was added thereto. The mixture was mixed, and thus a Part 2 was obtained.

The Part 1 was uniformly dispersed in the Part 2, and a mixture of a portion of component (1) and component (9) prepared by wet dispersing was added thereto. Thus, an intended O/W emulsion composition was obtained.

**[Table 1]**

| No. | Test Example | 1-1 | 1-2 |
|---|---|---|---|
| 1 | Water | Balance | Balance |
| 2 | Ethanol | 6 | 6 |
| 3 | Octocrylene | 5 | 5 |
| 4 | Ethylhexyl methoxycinnamate | 4.996 | 4.996 |
| 5 | 4-t-butyl-4'-methoxydibenzoylmethane | 2 | 2 |
| 6 | POE(20)POP(8) cetyl ether | 0.8 | 0.8 |
| 7 | Dipropylene glycol | 3 | 3 |
| 8 | Glycerin | 1 | 1 |
| 9 | Silica | 1 | 1 |
| 10 | Diisopropyl sebacate | 2 | 2 |
| 11 | Caprylyl methicone | 2 | 2 |
| 12 | Isostearic acid | 0.2 | 0.2 |
| 13 | Carbomer K | 0.131 | 0.131 |
| 14 | (Acrylic acid/alkyl acrylate(C10-30)) copolymer* | 0.066 | 0.066 |
| 15 | Trisodium edetate | 0.02 | 0.02 |
| 16 | BHT | 0.004 | 0.004 |
| 17 | Phenoxyethanol | 0.5 | 0.5 |
| | Production method | Production method 1 | Production method 2 |
| | Average emulsion particle size (nm) | 581 | 938 |
| | Emulsion stability | A | C |
| | Feeling in use (non-stickiness during application) | A | B |
| | Solubility stability | A | A |

| | | | |
|---|---|---|---|
| *: PEMULEN TR-2 (manufactured by BF Goodrich) | | | |

According to Table 1, it was found that if the emulsion particle size is large as in the case of Test Example 1-2, even in the same composition, the emulsion has a feeling of slight stickiness during the application on the skin, and emulsion stability is also insufficient.

As a result of the investigations made by the present inventors, it was clarified that when the average particle size of the emulsion particles is 800 nm or less, an O/W emulsion composition having excellent emulsion stability and usability can be obtained.

### TEST EXAMPLE 2 Amount of surfactant

O/W emulsion compositions were prepared in the same method with Test Example 1-1, except for changing the amounts of block polymer [POE(20)POP(8) cetyl ether] as an emulsifier. Then, each sample was evaluated in the above-described evaluation methods (1) to (4). The result is shown in Table 2.

**[Table 2]**

| Test Example | 1-1 | 2-1 | 2-2 |
|---|---|---|---|
| POE(20)POP(8) cetyl ether | 0.8 | 0.4 | 3.1 |
| Average emulsion particle size | A | A | A |
| Emulsion stability | A | B | A |
| Feeling in use (non-stickiness during application) | A | A | C |
| Solubility stability | A | A | A |

According to Table 2, it was found that the emulsion stability tends to decrease when the amount of block polymer is too small. When the block polymer is contained excessively, sticky feeling is caused during application.

From these results, the amount of block polymer is preferably 0.5 to 3 mass %, more preferably 0.5 to 2 mass %, and particularly preferably 0.5 to 1 mass %, in the O/W emulsion composition of the present invention.

According to the present invention, as shown in Table 2, it is possible that a large amount of oil-soluble organic UV absorber (e.g., 10 mass % or more) is stably emulsified with use of an extremely small amount of emulsifier (e.g., 1 mass % or less) to provide an O/W emulsion composition excellent in UV protection ability, formulation stability, and feeling in use.

### TEST EXAMPLE 3 Kind of surfactant

O/W emulsion compositions were prepared in the same method with Test example 1-1, except for changing the kinds of block polymer [POE(20)POP(8) cetyl ether] as an emulsifier. Then, each sample was evaluated in the above-described evaluation methods (1) to (4). The result is shown in Table 3.

**[Table 3]**

| Test Example | 1-1 | 3-1 | 3-2 | 3-3 | 3-4 | 3-5 | 3-6 | 3-7 | 3-8 | 3-9 | 3-10 |
|---|---|---|---|---|---|---|---|---|---|---|---|
| POE(20)POP(8) cetyl ether [HLB=12.5] | 0.8 | - | - | - | - | - | - | - | - | - | - |
| POE(50)POP(40) dimethyl ether [HLB=17] | - | 0.8 | - | - | - | - | - | - | - | - | - |
| POE(35)POP(40) dimethyl ether [HLB=12] | - | - | 0.8 | - | - | - | - | - | - | - | - |
| POE(30) behenyl ether [HLB=18] | - | - | - | 0.8 | - | - | - | - | - | - | - |
| POE(60) glyceryl isostearate [HLB=18.3] | - | - | - | - | 0.8 | - | - | - | - | - | - |
| POE(60) hydrogenated castor oil [HLB=14.6] | - | - | - | - | - | 0.8 | - | - | - | - | - |
| POE(100) hydrogenated castor oil [HLB=16.5] | - | - | - | - | - | - | 0.8 | - | - | - | - |
| POE(30) phytosterol [HLB=18] | - | - | - | - | - | - | - | 0.8 | - | - | - |
| POE(30) cholestanol [HLB=17] | - | - | - | - | - | - | - | - | 0.8 | - | - |
| POE(20) sorbitan monostearate [HLB=14.9] | - | - | - | - | - | - | - | - | - | 0.8 | - |
| POE(20) sorbitan monolaurate [HLB=16.9] | - | - | - | - | - | - | - | - | - | - | 0.8 |
| Average emulsion particle size | A | A | C | C | C | C | C | C | C | C | C |
| Emulsion stability | A | A | B | B | B | B | B | B | B | C | C |
| Feeling in use (non-stickiness during application) | A | A | B | B | B | B | B | B | B | C | C |
| Solubility stability | A | A | A | A | A | A | A | A | A | A | A |

According to Table 3, with polyoxyethylene/polyoxyalkylene alkyl ether block polymer of the Test Example 1-1 or 3-1, it was found that the emulsion particles containing the component (a) could be easily emulsified to be 800 nm or less to obtain the O/W emulsion composition excellent in formulation stability and feeling in use could be obtained. However, with polyoxyethylene/polyoxyalkylene alkyl ether block polymer of the Test Example 3-2 or the other nonionic surfactants, even when the surfactant having HLB in the same range was used, it was difficult to obtain such an O/W emulsion composition.

### TEST EXAMPLE 4 Average addition mole number of EO and PO of polyoxyethylene/polyoxyalkylene alkyl ether block polymer

An investigation was conducted on the details of the polyoxyethylene/polyoxyalkylene alkyl ether block polymer which is capable of emulsifying emulsion particles containing the component (a) to be 800 nm or less.

O/W emulsion compositions were prepared in the same method with Test example 1-1, except for changing average addition mole number m of EO and/or average addition mole number n of PO of block polymer [POE(20)POP(8) cetyl ether] (C₁₆H₃₃O-(PO)m-(EO)n-H) as an emulsifier. Then, each sample was evaluated in the above-described evaluation method (1). The result is shown in Table 4.

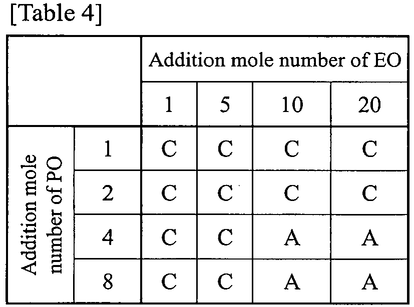

According to Table 4, it was clarified that when the addition mole number of EO is less than 10 or the addition mole number of PO is less than 4, the average particle size of the emulsion particles of the sample exceeds 800 nm. Furthermore, as a result of a new study, it was clarified that when the addition mole number of EO or the addition mole number of PO is 70 or more, handleability is deteriorated.

From these results, it is necessary that a polyoxyethylene/polyoxyalkylene alkyl ether block polymer represented by formula R₁O-(PO)m-(EO)n-H satisfies 4≤m<70, 10≤n<70, and m< n.

### TEST EXAMPLE 5 Blending ratio of 4-t-butyl-4'-methoxydibenzoylmethane

O/W emulsion compositions were prepared in the same method with Test example 1-1, except for changing the amount of 4-t-butyl-4'-methoxydibenzoylmethane. Then, each sample was evaluated in the above-described evaluation method (4). The result is shown in Table 5.

**[Table 5]**

| Test Example | 5-1 | 1-1 | 5-2 |
|---|---|---|---|
| Octocrylene | 5 | 5 | 5 |
| Ethylhexyl methoxycinnamate | 4.996 | 4.996 | 4.996 |
| 4-t-butyl-4'-methoxydibenzoylmethane | 0.5 | 2 | 5.1 |
| (a1)+(a2):(a3) | 19.99:1 | 4.998:1 | 1.961:1 |
| Solubility stability | A | A | B |

According to Table 5, when 4-t-butyl-4'-methoxydibenzoylmethane is incorporated into an oil phase containing octocrylene and ethylhexyl methoxycinnamate, 4-t-butyl-4'-methoxydibenzoylmethane can be dissolved in a large amount.

However, as in the case of Test Example 5-2, if the mixing ratio of octocrylene and ethylhexyl methoxycinnamate to 4-t-butyl-4'-methoxydibenzoylmethane is higher than 2:1, the solubility gradually becomes poorer.

Accordingly, in the present invention, the value of {amount of (a1) + amount of (a2)}/amount of (a3) is preferably 2 or more and particularly preferably 4.5 or more.

### TEST EXAMPLE 6 Amount of organic UV absorber

O/W emulsion compositions were prepared in the same method with Test example 1-1, except for changing the amounts of organic UV absorbers. Then, each sample was evaluated in the above-described evaluation methods (1) to (5). The result is shown in Table 6.

**[Table 6]**

| Test Example | 6-1 | 6-2 | 5-1 | 1-1 | 6-3 | 5-2 | 6-4 |
|---|---|---|---|---|---|---|---|
| Octocrylene | 1 | 5 | 5 | 5 | 5 | 5 | 10.1 |
| Ethylhexyl methoxycinnamate | 4.996 | 1 | 4.996 | 4.996 | 7.6 | 4.996 | 4.996 |
| 4-t-butyl-4'-methoxydibenzoylmethane | 2 | 2 | 0.5 | 2 | 2 | 5.1 | 2 |
| Total amounts of organic UV absorbers | 7.996 | 8 | 10.496 | 11.996 | 14.6 | 15.096 | 17.096 |
| Average emulsion particle size | A | A | A | A | A | A | A |
| Emulsion stability | A | A | A | A | B | B | B |
| Feeling in use (non-stickiness during application) | A | A | A | A | B | B | B |
| Solubility stability | C | C | A | A | A | B | A |
| UV protection ability | C | C | C | A | A | A | A |

According to Table 6, all the samples in which various amounts of organic UV absorbers were incorporated, had an average emulsion particle size of 800 nm or less (for example, the size was 483 nm in Test Example 5-1, and 522 nm in Test Example 5-2).

However, as in the cases of Test Examples 6-1, 6-2, and 5-1, if the amounts of the organic UV absorbers were small, a composition having satisfactory emulsion stability was produced, but it had a poor UV protection ability.

Furthermore, it was found that, as in the cases of Test Examples 6-3, 6-4, and 5-2, if the organic UV absorbers were contained excessively, stickiness and a decrease in emulsion stability were brought about.

Accordingly, in the O/W emulsion composition of the present invention, it is preferable that the amount of octocrylene is 10 mass % or less, the amount of ethylhexyl methoxycinnamate is 7.5 mass % or less, and the amount of 4-t-butyl-4'-methoxydibenzoylmethane is 5 mass % or less.

Also, considering UV protection ability, the total amounts of organic UV absorbers are preferably 8 mass % or more, more preferably 9 mass % or more, and particularly preferably 10.5 mass % or more.

## Claims

1. An O/W emulsion composition comprising the following components (a) and (b):
(a) organic UV absorbers comprising the following components (a1), (a2), and (a3):
(a1) octocrylene,
(a2) ethylhexyl methoxycinnamate,
(a3) 4-t-butyl-4'-methoxydibenzoylmethane; and
(b) a polyoxyethylene/polyoxyalkylene alkyl ether block polymer represented by the following formula (1);
(Compound 1)
R₁O-(PO)m-(EO)n-H (1)
wherein R₁ is a hydrocarbon group having 16 to 18 carbon atoms; PO is an oxypropylene group, EO is an oxyethylene group, and PO and EO are added to each other in block form; and m and n respectively represent average addition mole number of PO and EO, 4≤m<70, 10≤n<70, and m< n;
wherein average particle size of the emulsion particles comprising the component (a) is 800 nm or less, and
wherein the value of {amount of (a1) + amount of (a2)}/amount of (a3) is 2 or more.

2. The O/W emulsion composition according to claim 1, wherein the amount of component (a) is 8 mass % or more.

3. The O/W emulsion composition according to claim 1 or 2, wherein the amount of component (b) is 0.5 to 3 mass %.

4. The O/W emulsion composition according to any of claims 1 to 3, wherein the amount of component (a1) is 1 to 10 mass %, the amount of component (a2) is 1 to 7.5 mass %, and the amount of component (a3) is 0.5 to 5 mass %.

5. A sunscreen cosmetic consisting of the O/W emulsion composition according to any of claims 1 to 4.

## Patentansprüche

1. Öl-Wasser-Emulsionszusammensetzung, enthaltend die folgenden Bestandteile (a) und (b):
(a) organische UV-Absorber, enthaltend die folgenden Bestandteile (a1), (a2) und (a3)
(a1) Octocrylen,
(a2) Ethylhexylmethoxycinnamat; und
(a3) 4-t-butyl-4'-methoxydibenzoylmethan; und
(b) ein Polyoxyethylen/Polyoxyalkylen-Alkylether-Blockpolymer, dargestellt durch die folgende Formel (1);
(Verbindung 1)
R₁O-(PO)m-(EO)n-H (1)
wobei R₁ eine Kohlenwasserstoffgruppe mit 16 bis 18 Kohlenstoffatomen ist; PO eine Oxypropylengruppe ist, EO eine Oxyethylengruppe ist und PO und EO einander in Blockform zugegeben werden; und wobei m und n jeweils eine durchschnittliche Zugabe-Molzahl von PO und EO sind, wobei gilt: 4≤m<70, 10≤n<70 und m<n;
wobei die durchschnittliche Partikelgröße der Emulsionspartikel, die den Bestandteil (a) enthalten, 800 nm oder kleiner ist und
wobei der Wert der {Menge von (a1) + Menge von (a2)}/Menge von (a3) gleich 2 oder höher ist.

2. Öl-Wasser-Emulsionszusammensetzung nach Anspruch 1, wobei die Menge des Bestandteils (a) 8 Masse-% oder mehr beträgt.

3. Öl-Wasser-Emulsionszusammensetzung nach Anspruch 1 oder 2, wobei die Menge des Bestandteils (b) 0,5 bis 3 Masse-% beträgt.

4. Öl-Wasser-Emulsionszusammensetzung nach einem der Ansprüche 1 bis 3, wobei die Menge des Bestandteils (a1) 1 bis 10 Masse-%, die Menge des Bestandteils (a2) 1 bis 7,5 Masse-% und die Menge des Bestandteils (a3) 0,5 bis 5 Masse-% beträgt.

5. Sonnenschutzkosmetik, bestehend aus der Öl-Wasser-Emulsionszusammensetzung nach einem der Ansprüche 1 bis 4.

## Revendications

1. Composition d'émulsion h/e comprenant les composants suivants (a) et (b):
(a) des absorbeurs UV organiques comprenant les composants suivants (a1), (a2) et (a3):
(a1) l'octocrylène,
(a2) le méthoxycinnamate d'éthylhexyle
(a3) le 4(1-t-butyl-4'-méthoxydibenzoylméthane); et
(b) un copolymère bloc d'éthers d'alkyle de polyoxyéthlène/polyoxyalkylène représenté par la formule suivante (1);
(Composé 1)
R₁O-(PO)m-(EO)n-H (1)
dans laquelle R₁ représente un groupe hydrocarboné comprenant de 16 à 18 atomes de carbone; PO représente un groupe oxypropylène, EO représente un groupe oxyéthylène, et PO et EO sont ajutés les uns aux autres sous forme de bloc; et m et n respectivement représentent des nombres de moles de produit d'addition moyens de PO et EO, 4≤m<70, 10≤n<70 et m<n;
la taille particulaire moyenne des particules d'émulsion comprenant le composant (1) étant 800 nm ou moins, et
la valeur de {la quantité d'(a1) + la quantité d'(a2)}/la quantité d'(a3) est 2 ou plus.

2. Composition d'émulsion h/e selon la revendication 1, dans laquelle la quantité du composant (a) est 8% en masse ou plus.

3. Composition d'émulsion h/e selon la revendication 1 ou 2, dans laquelle la quantité (b) est 0,5 à 3% en masse.

4. Composition d'émulsion h/e selon l'une des revendications 1 à 3, dans laquelle la quantité du composant (a1) est 1 à 10% en masse, la quantité du composant (a2) est 1 à 7,5% en masse, et la quantité du composant (a3) est 0,5 à 5% en masse.

5. Préparation cosmétique écran solaire constituée d'une composition d'émulsion h/e selon l'une des revendications 1 à 4.
